# EUROPEAN PATENT SPECIFICATION

(11) **EP 4 633 522 B1**
(45) Date of publication and mention of the grant of the patent: **11.11.2026**
(21) Application number: 23840777.9
(22) Date of filing: 14.12.2023
(51) Int. Cl.: A61B 90/00, G16H 40/63

(54) **RESISTANCE LEVEL COMMUNICATION PROTOCOL**
ÜBERTRAGUNGSPROTOKOLL BASIEREND AUF WIDERSTANDSWERTEN
PROTOCOLE DE COMMUNICATION DE NIVEAU DE RÉSISTANCE

(30) Priority: 16.12.2022 US 202218083287
(43) Date of publication of application: 22.10.2025
(73) Proprietor: Medtronic Advanced Energy LLC, Minneapolis, MN 55432 (US)
(72) Inventor: MIRI, Mohammad, Lafayette, Colorado 80026 (US)
(74) Representative: Viering, Jentschura & Partner mbB Patent- und Rechtsanwälte
(86) International application number: PCT/IB2023/062670
(87) International publication number: WO 2024/127291

(56) References cited:
- US-A1- 2012 265 196
- US-A1- 2019 021 783
- US-A1- 2020 090 808
- US-A1- 2021 315 582

## Description

### FIELD

This application includes subject matter related to U.S. Pat. App. No. 18/083,287.

Embodiments of the present disclosure relate generally to the field of electrosurgical energy delivery, and more particularly to a resistance level communication protocol for communication between an electrosurgical handpiece and an electrosurgical generator.

### BACKGROUND

Electrosurgical devices for applying electrical energy to tissue are commonly used in surgical procedures for hemostatic sealing and coagulation of soft tissue and bone at the operative site. Such electrosurgical devices can be used for, but not limited to orthopedic, spine, thoracic, and open abdominal surgery.

An electrosurgical device may comprise a hand piece having a distally mounted end comprising one or more electrodes. The one or more electrodes can be positioned against the tissue such that electrical current is introduced into the tissue. The generated heat can be used to cut, coagulate or induce metabolic processes in the target tissue. An electrosurgical generator generally provides power and electrical energy in the form of radio frequency ("RF") energy to one of two handpiece topologies, the monopolar and the bipolar.

The handpiece can include one or more buttons for energy activation. Generally the internal digital circuit in the electrosurgical generator is isolated from the front end of the generator and consequently the generator uses a low frequency signal with an isolation transformer to detect the button status of the handpiece. Conventional approaches to powering the handpiece often use a specific level of resistance to detect high resistance, indicating the button is released, and low resistance, indicating the button is pressed.

Conventional electrosurgical handpieces used for electrosurgical tissue treatment are not able to establish communication with the electrosurgical generator and accordingly face several challenges. Some challenges that arise include an inability to determine the manufacturer and model of the electrosurgical handpiece as well as the usage time. Understanding device specific information about an electrosurgical handpiece coupled to a generator is beneficial for safety and consistent treatment delivery.

Accordingly, improved systems and methods are desired for enhancing the communication capabilities of electrosurgical device without changing the generator or requiring a second electrical connection to the electrosurgical device.

In this regard, US 2012/265196 A1 discloses a control circuit of a surgical device, wherein the control circuit includes a first circuit portion coupled to at least one switch operable between an open state and a closed state, wherein the first circuit portion communicates with a surgical generator over a conductor pair to receive a control signal to determine a state of the at least one switch. Further electrosurgical devices and related techniques are disclosed in US 2019/021783 A1, US 2021/315582 A1 and US 2020/090808 A1.

### SUMMARY

The techniques of this disclosure generally relate to an electrosurgical device configured to communicate with an electrosurgical generator using existing button press signal to enact a resistance level communication protocol, so as to increase functionality of the electrosurgical device without requiring any change to wiring of an electrosurgical generator.

To this end, the present invention provides systems as set out in claims 1 and 14, and a method as set out in claim 7. Further advantageous embodiments are described in the dependent claims.

Accordingly, in one aspect, the present disclosure provides a system configured to establish two-way communication using a resistance level communication protocol. The system includes a generator and an electrosurgical device. The electrosurgical device comprises an electrical connector configured to receive a treatment signal and a continuous signal from the generator, an AC-DC converter configured to convert the continuous signal to a DC signal, a processor configured to receive the DC signal, and a resistor connected to the processor and configured to vary a current of power used by the processor over time. The generator is configured to interpret variances in the current as data and provide an acknowledgement to the electrosurgical device using frequency shift keying (FSK).

In another aspect, the disclosure provides a method of two-way communication between an electrosurgical device and a generator. The method comprises providing, via the generator, a continuous signal to the electrosurgical device. Then, at the electrosurgical device, converting the continuous signal to a DC signal, powering a processor with the converted signal, varying, using a resistor, the current of power used by the processor over time, and providing the current to the generator. The method further comprises, at the generator, interpreting variances in the altered current as data and sending an acknowledgement to the electrosurgical device using frequency shift keying (FSK).

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Subject matter hereof may be more completely understood in consideration of the following detailed description of various embodiments in connection with the accompanying figures, in which
FIG. 1 is a schematic view depicting an electrosurgical device in accordance with the prior art.
FIG. 2 is a schematic view depicting an electrosurgical device according to an embodiment.
FIG. 3 is a diagram of resistance over time of an electrosurgical device circuit according to an embodiment.
FIG. 4 is a diagram of resistance over time of an electrosurgical device circuit represented as binary values according to an embodiment.
FIG. 5 is a schematic view depicting an electrosurgical device according to an embodiment.
FIG. 6 is a schematic view depicting an electrosurgical device according to an embodiment.
FIG. 7 is a schematic view depicting an electrosurgical device according to an embodiment.
FIG. 8 is a flowchart of a method for interactive encryption according to an embodiment.
FIG. 9 is a flowchart of a method for two-way communication between an electrosurgical device and a generator, according to an embodiment.

While various embodiments are amenable to various modifications and alternative forms, specifics thereof have been shown by way of example in the drawings and will be described in detail. It should be understood, however, that the intention is not to limit the claimed inventions to the particular embodiments described. On the contrary, the intention is to cover the subject matter as defined by the claims.

### DETAILED DESCRIPTION

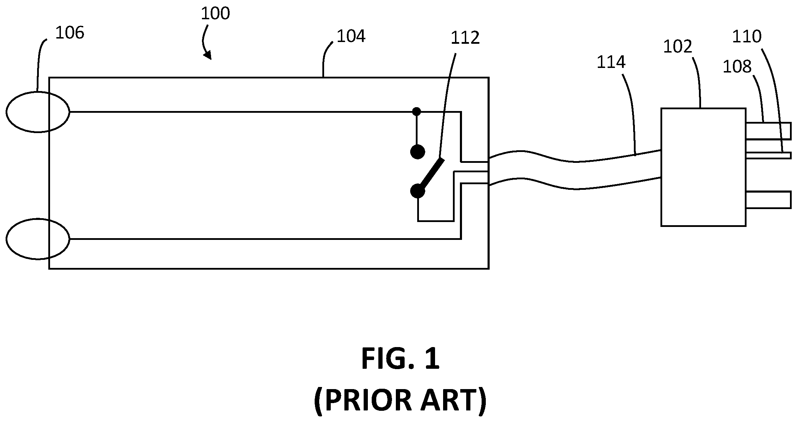

FIG. 1 is a partial schematic diagram of an electrosurgical device 100 configured to transmit RF energy at a treatment site to provide hemostatic sealing and coagulation of soft tissue and bone. Electrosurgical device 100 includes connector 102 and bipolar handpiece 104 having two electrodes 106.

Connector 102 includes large pins 108 and small pin 110 and is configured to be in electrical communication with an electrosurgical generator (not pictured) and a proximal end of bipolar handpiece 104 such that power signals are delivered to electrodes 106 at a distal end of bipolar handpiece 104. In embodiments, connector 102 can be a 3-pin connector capable of providing a high-power treatment signal and a low-power continuous signal via cable 114. Large pins 108 are for a high-power treatment signal and small pin 110 uses a low-power continuous signal to detect a button press on the handpiece. When the button is pressed, the circuit is closed by switch 112 and the treatment signal is provided. In embodiments, an example of the treatment signal is at 469KHz and 20W to 220W while an example of continuous signal is 47KHz.

It will be appreciated that the terms "proximal" and "distal" are used herein with reference to a clinician gripping a handpiece. Thus, electrodes 106 are distal with respect to the more proximal handle or gripping portion of bipolar handpiece 104. However, surgical devices are used in many orientations and positions, and these terms are not intended to be limiting and absolute.

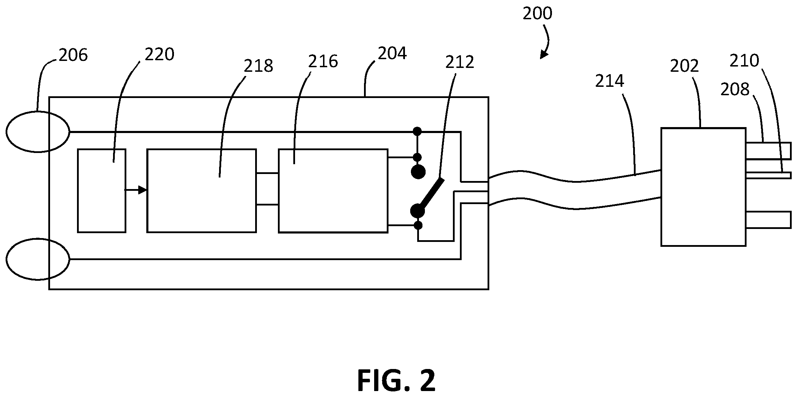

FIG. 2 depicts a schematic diagram of an electrosurgical device 200 configured to use the RF signal used for detecting a button press to communicate device data to an electrosurgical generator. Communicated device data can include one or more of a device ID, accumulated use time for the device, manufacturing information, and waveform information.

Electrosurgical device 200 includes connector 202 and bipolar handpiece 204 having two electrodes 206, switch 212, cord 214, transformer 216, digital potentiometer 218, and controller 220. Connector 202 includes large pins 208 and small pin 210 and is configured to be compatible with existing electrosurgical power source or bipolar energy supplies. For example, the transcollation sealing energy supplied by the Aquamantys^{®} System (available from Medtronic Advanced Energy of Portsmouth, N.H.) may be used. U.S. Pat. Nos. 6,558,385; 6,702,810, 6,953,461; 7,115,139, 7,311,708; 7,537,595; 7,645,277 and 7,811,282 also describe bipolar ablation energy systems suitable for use with embodiments of the present disclosure.

Accordingly, electrosurgical device 200 connects to a source of electrical energy via connector 202. Because connector 202 is designed for compatibility with preexisting generators, the prongs are standardized based on the desired generator. In embodiments, more or less large prongs and short prongs may be used depending on the requirements of the generator. In an embodiment, small pin 210 is used to detect the button activation. Button press detection can occur from monitoring resistance levels from a low power signal produced by the electrosurgical generator. For example, the electrosurgical generator can inject a 47KHz low power signal between one of the large pins 208 and the small pin 210 and continuously monitor the resistance between these two pins (e.g., every 1msec). If the resistance is detected to be anything less than a threshold resistance the electrosurgical generator can determine that the button pressed and switch 212 is closed, otherwise the button can be determined to be released and switch 212 is open. In an embodiment the threshold resistance is 400 ohm.

The current reading at the generator is not linear with the resistance threshold. Accordingly, an appropriate resistance threshold should be determined based on the minimum resistance.

In embodiments, the generator can detect the resistance between a first pin connected to one side of a push button and a second pin connected to the other side of the push button. In such embodiments the third pin would be connected to an electrode 206. An analog-to-digital converter (ADC) inside the generator is configured to convert detected resistance to digital counts (*e.g.* for a 12bit ADC the conversion would be 0 to 4095 counts). A processor of the generator is then configured to consider any reading equal to or higher than a threshold count as a pressed button and any reading less than the threshold count considered released button. Counts represent the current through the button so a lower resistance results in higher counts. The threshold counts is defined as Rₘᵢₙ. In embodiments, Rₘᵢₙ can be 1000 count. In such embodiments, each reading between 0 count and 999 count can be considered an open circuit, although the actual number can be available.

The addition of a parallel resistor such as digital potentiometer 218 isolated by transformer 216 effectively makes the overall resistance between the first pin and second pin a combination of the two resistances. The overall resistance can be represented as: ${R}_{total} = \frac{{R}_{B} ⋅ {R}_{DP}}{\left({R}_{B}+{R}_{DP}\right)}$

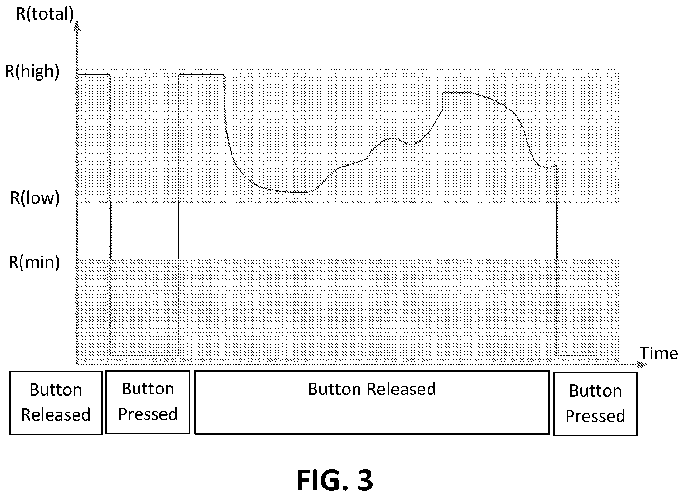

In this arrangement when a button is pressed Rₜₒₜₐₗ is effectively 0, making button press detection virtually equivalent to an electrosurgical device without the additional circuit. However, when the button is released, Rₜₒₜₐₗ is equal to R_{DP}. If R_{DP} adjusted to less than Rₘᵢₙ, the generator will activate the handpiece without pressing the button. Therefore, anything less than Rₘᵢₙ should be avoided for the digital potentiometer to prevent unintentional activation of the electrosurgical device. Practically, a margin should be added to Rₘᵢₙ to ensure user safety. Accordingly, R_{low} can be equal to the sum of Rₘᵢₙ and R_{margin} while R_{high} is the max resistance that can be adjusted by the digital potentiometer. As a result a diagram of Rₜₒₜₐₗ over time can be configured as depicted in FIG. 3.

When the button is released, the generator will be able to detect the values between R_{low} and R_{high} and if the value of the R_{DP} is coded by controller 220 with a digital message, the generator will be able to receive a message.

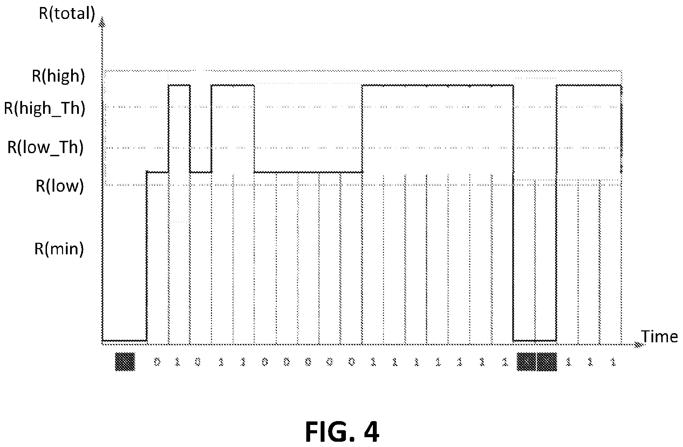

Referring to FIG. 4, a diagram depicts the changes in the resistance over time of a digital potentiometer, such as digital potentiometer 218 corresponding to digital data. In embodiments, when a button of the electrosurgical device is pressed, the generator resistance reading is undefined from the communication aspect and the resistance will only be considered as button detection.

Although the rise and fall of resistance theoretically happens immediately as shown in FIG. 4, practically the value of the digital potentiometer cannot change immediately and a rise and fall time must be accounted for. In embodiments, a third of the resistance range (R_{low}-R_{high}) from the highest resistance will be considered as digital high threshold, R_{High_Th}, and a third of the resistance range from the bottom is considered the digital low threshold, R_{Low_Th}. The value between R_{High_Th}, and R_{High_Low}, can be considered undefined. In embodiments, up to half of the resistance range can be used as digital thresholds for interpreting communications. However, it is generally preferable to have some resistance range be undefined to better distinguish between digital data. In an embodiment, R_{High_Th} can be a fourth of the resistance range lower than R_{High} while R_{Low_Th} can be a fourth of the resistance range higher than R_{Low}.

To prevent the occurrence of an undefined state, the communication protocol can allow enough time for resistance levels to vary between a high level and a low level. The stack code of the communication protocol can consider multiple readings within a time period to assure the level is stable. In embodiments the time period can be defined as 10 times higher than rise and fall time (whichever is higher) and will depend on the component used for the digital potentiometer.

Over time the controller within the electrosurgical device can change resistance of the digital potentiometer in a pattern to communicate device data. In embodiments, the communicated device data can include a pattern representing device ID and then accumulated use time. The range of reading must take into account the unauthorized zone to prevent unintentional activation. If R_{DP} is adjusted to less than Rₘᵢₙ, the generator will activate the handpiece without pressing the button. Therefore anything less than Rₘᵢₙ is an unauthorized zone for the digital potentiometer.

The resistance level communication protocol of the present disclosure accordingly allows for a peer-to-peer single sided communication hardware protocol by translating the resistance of the button press signal to digital data without affecting the main functionality of the wires being used. Notably, the definition of resistance level communication protocol is not only limited to using the different resistance changes when the main functionality is considering the resistance open. This method is only one way of using this protocol by the hardware parallel digital potentiometer topology. For example, in embodiments different resistance changes can be used when the main functionality is considering the resistance closed. Additionally alternate actuation controls such as a slider, switch, or other input mechanisms can be used in place of a button to open and close the circuit.

Embodiments of the present disclosure therefore provide for a resistance level communication protocol to enable communication between an electrosurgical handpiece and an electrosurgical generator. As a result of this communication, the electrosurgical generator can receive data from the electrosurgical handpiece in order to recognize the type of device connected to the bipolar port and other device information. This device recognition can allow the electrosurgical generator to prevent operation if the electrosurgical device is not recognized, improving safety by restricting use to known equipment and operating parameters.

The resistance level communication protocol can effectively be accomplished without changes to the hardware of the electrosurgical generators and with only minimal changes to the handpiece. The hardware changes to the electrosurgical handpiece introduce no new wiring coming from electrosurgical device 200 so as to not be cumbersome in operation and storage. Further, because no hardware changes are necessary to the electrosurgical generator, the resistance level communication protocol can be extended to preexisting electrosurgical generators via a software update.

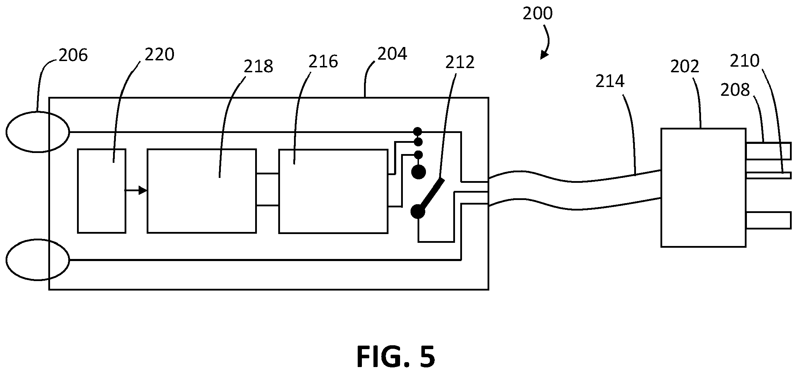

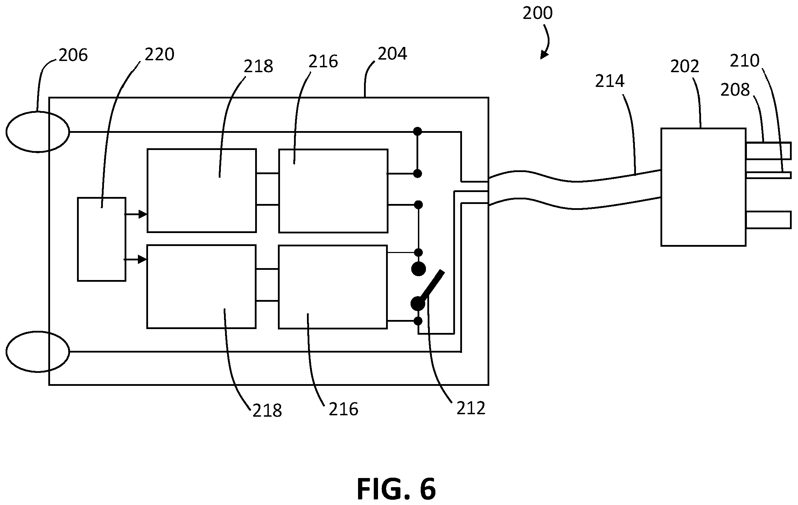

Alternate arrangements of electrosurgical device 200 are depicted in FIGS. 5 and 6. These arrangements introduce a digital potentiometer in series with the main line and main functionality device and utilize the lower side of the resistance. These arrangements can be used if the majority of the time, the main application represents a short circuit. For electrosurgical handpieces or devices where the button is pressed for the majority of the time, it can be more reasonable to use the range of pressed button for the ADC inside the generator to detect the communication as this will enable longer periods for device data be communicated. Placing the digital potentiometer in series, as shown in FIG. 5, can make such communication possible. FIG. 6 depicts a hybrid mode of resistance communication level protocol by having two digital potentiometers with one controller to take advantage of communication when the button is pressed and released.

In embodiments, controller 220 can be powered from button signal impedance measured by measuring current rather than resistance. Such embodiments can allow for increased power of microcontroller recording and a single resistor by controller 220 such that no isolation transformer is needed. The frequency range that can be used to communicate device data is comparatively limited if no other components are included with the controller in the circuit. This reduction in range is due to added resistance by the controller. In such embodiments the reduced frequency range is sufficient to be operable with the resistance level communication protocol described by the present disclosure.

One drawback of single sided communication is that the communication signal can be replicated by a different circuit, introducing security concerns. To address this concern, encryption can be implemented from the electrosurgical handpiece to the generator but that first requires a handshake (two way communication). The inventors of the present disclosure have discovered a means of creating a communication, that can be used as handshake for resistance level communication protocol, from the generator to the electrosurgical device.

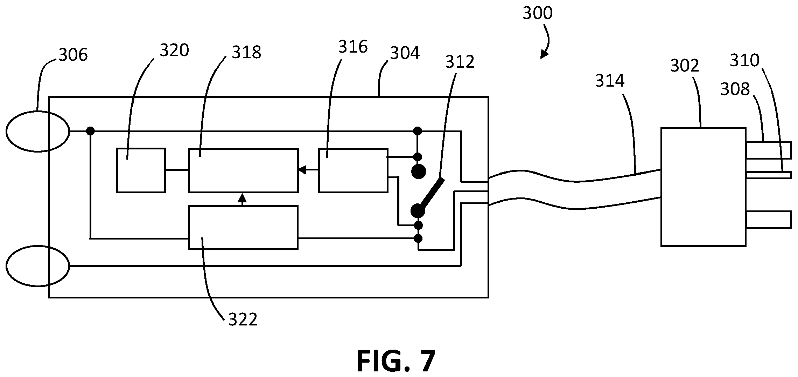

FIG. 7 depicts a schematic diagram of an electrosurgical device 300 configured to facilitate two-way communication with an electrosurgical generator. Electrosurgical device 300 includes connector 302 and bipolar handpiece 304 having two electrodes 306, switch 312, cord 314, AC-DC converter 316, processor 318, and resistor 320. Connector 302 includes large pins 308 and small pin 310 and is configured to be compatible with existing electrosurgical power source or bipolar energy supplies.

As previously described, the generator uses a signal to detect when the button is pressed and this is the same signal that is used for resistance level communication protocol DC power (extracted energy) and for resistance measurement. Both the generator and the electrosurgical device 300 do not rely on the frequency of the button signal, meaning that small changes in frequency to the button detect signal would not alter the functionality of the button detect, the resistance level communication protocol power supply, and the resistance level communication protocol. For example, if the generator typically uses a 47KHz signal to detect the button press that frequency can be changed to 40KHz instead of 47KHz without interrupting operation. The circuit should be checked for the cutoff frequency to determine the range at which the button detect frequency can be manipulated without effecting operation.

The button press detection frequency is generated inside the generator from a complex programming logic device (CPLD) or field-programmable gate array (FPGA). A processor is in continuous communication with CPLD and can notify the CPLD to use a different frequency than the standard operating frequency. Implementation of a variance in button press detection frequency can accordingly be changed through a software update, making two-way communication possible using preexisting generators.

The technique of sending digital data by changing frequency is called Frequency Shift Keying (FSK). The FSK receiver can be a simple counter that can measure the frequency and convert that into data. The counter can be as simple as one pin of processor 318 with shaping (clipping) circuit 322 to convert the sine wave to square wave. With this method, two-way communication can be stablished.

Highs and Lows on resistor 320 change the current on the power consumption of processor 318 which comes from AC-DC converter 316. The result is those Highs and Lows can be translated into current being sensed in the generator to implement resistance level communication protocol.

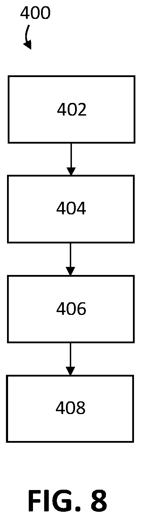

An interactive encryption in the acknowledgement message from the generator can be implemented with electrosurgical device 300. FIG. 8 depicts a flowchart of a method 400 for such interactive encryption according to an embodiment. Method 400 applies a function to a device ID of the electrosurgical device to create encrypted data. Because the encryption method and encryption output are dependent on the device ID and will therefore be varied.

At 402, a first potion (*e.g.* the first 8 bits) of the device ID is read. In embodiments, different device-specific static data may be used in place of a device ID.

At 404, the first portion of the device ID can be used to define what encryption algorithm will be used.

At 406, the selected encryption algorithm is applied to the device ID.

At 408, the output of the selected encryption algorithm from 406 is cut to fit in a reduced number of Bytes. In embodiments, the output is cut to fit in 2 Bytes. This size reduction introduces the possibility that from the encrypted data, the plain data would not be unique. Since the goal for this encryption is to acknowledge a known plain data, it is beneficial to make it nonrecoverable.

Method 400 can output plain data that is known by both the electrosurgical device and the generator. This simplifies the handshake to a simple comparison of the output from each device. Method 400 therefore provides for double encrypted data that is interactively tied to the plain data as the method of encryption is itself also encrypted.

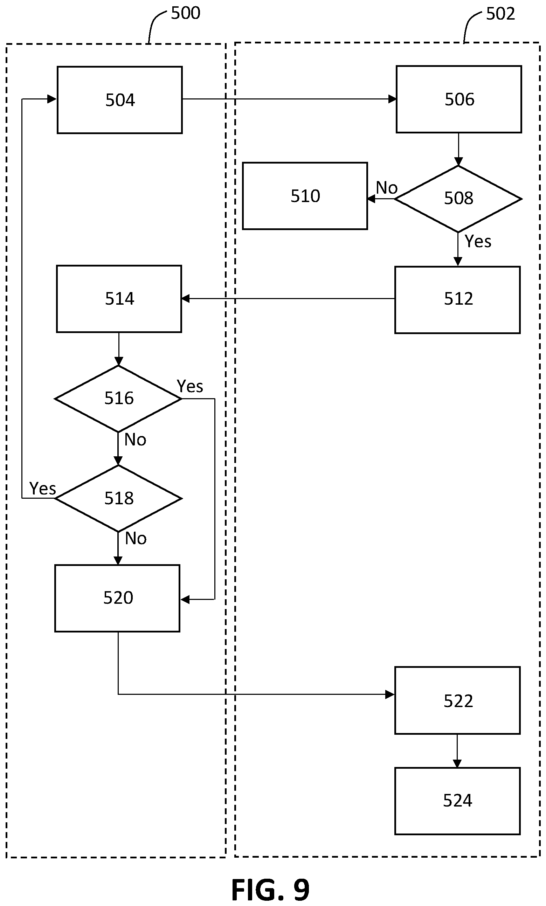

Referring to FIG. 9, a flowchart of electrosurgical device operations 500 and generator operations 502 to implement method 400 are depicted according to an embodiment.

At 504 the electrosurgical device sends the device ID to the generator using the resistance level communication protocol.

At 506 the generator receives the device ID over the resistance level communication protocol.

At 508 the generator determines if the device ID is correct.

If the device ID is not correct, at 510 an error is displayed or otherwise reported to a user.

If the device ID is correct, at 512 an encrypted response and real-time communication (RTC) are sent by the generator to the electrosurgical device using FSK. Sending this information serves as an acknowledgement of the device ID.

At 514 the electrosurgical device receives the acknowledgement.

At 516 the electrosurgical device determines whether the electrosurgical device is expired.

If the electrosurgical device is not expired, at 518 the electrosurgical device determines whether the acknowledgement is correct. If the acknowledgement is correct, the electrosurgical device returns to 504 and continues to report device data.

If the electrosurgical device is expired, at 520 the electrosurgical device intentionally sends an incorrect device ID to the generator using the resistance level communication protocol.

At 522 the generator receives the incorrect device ID over the resistance level communication protocol.

At 524 an error is displayed or otherwise reported to a user. In embodiments the incorrect device ID can be a particular code configured to result in a unique error such that a user may be made aware the electrosurgical device is expired.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A system configured to establish two-way communication using a resistance level communication protocol, the system comprising:
a generator; and
an electrosurgical device (300) comprising:
an electrical connector (302) configured to receive a treatment signal and a continuous signal from the generator;
an AC-DC converter (316) configured to convert the continuous signal to a DC signal;
a processor (218) configured to receive the DC signal; and
a resistor (320) connected to the processor (318) and configured to vary a current of power used by the processor (318) over time;
wherein the generator is configured to interpret variances in the current as data and provide an acknowledgement to the electrosurgical device (300) using frequency shift keying, FSK.

2. The system of claim 1, wherein the acknowledgment is based on the data,
wherein, optionally, the acknowledgement comprises output of an encryption algorithm applied to the data,
wherein, further optionally, the encryption algorithm is selected based on at least a portion of the data.

3. The system of claim 1, wherein the acknowledgment is a set number of bytes.

4. The system of claim 1, wherein the data is based on multiple readings of the current within a time period.

5. The system of claim 1, wherein the generator is further configured to limit the frequency of the treatment signal based on the data.

6. The system of claim 1, wherein the generator is further configured to provide an alert that the electrosurgical device (300) has expired based on the data.

7. A method of two-way communication between an electrosurgical device and a generator comprising:
providing, via the generator, a continuous signal to the electrosurgical device (300) and at the electrosurgical device (300):
converting the continuous signal to a DC signal;
powering a processor (318) with the DC signal; and
varying, using a resistor (320), the current of power used by the processor (318) over time;
providing, via the electrosurgical device (300), the current to the generator and at the generator:
interpreting variances in the current as data; and
sending an acknowledgement to the electrosurgical device (300) using frequency shift keying, FSK.

8. The method of claim 7, wherein the data is one or more of a device ID, an accumulated use time for the device, manufacturing information, and waveform information.

9. The method of claim 7, wherein the acknowledgment is based on the data,
wherein, optionally, the acknowledgement comprises output of an encryption algorithm applied to the data,
wherein, further optionally, the encryption algorithm is selected based on at least a portion of the data.

10. The method of claim 7, wherein the acknowledgment is a set number of Bytes.

11. The method of claim 7, wherein the data is based on multiple readings of the current within a time period.

12. The method of claim 7, wherein the generator is further configured to limit the frequency of a treatment signal provided to the electrosurgical device (300) based on the data.

13. The method of claim 7, further comprising the generator providing an alert that the electrosurgical device (300) has expired based on the data.

14. A system configured to establish two-way communication using a resistance level communication protocol, the system comprising:
a generator; and
an electrosurgical device (200) comprising:
an electrical connector (202) configured to receive a treatment signal and a continuous signal from the generator;
a transformer (216) configured to isolate the continuous signal;
a digital potentiometer (218) configured to receive the isolated continuous signal; and
a controller (220) configured to vary the resistance of the digital potentiometer over time;
wherein the generator is configured to interpret the variation in the resistance as data and provide an acknowledgement to the electrosurgical device (200) using frequency shift keying, FSK.

15. The system of claim 1 or of claim 14, wherein the data is one or more of a device ID, an accumulated use time for the device, manufacturing information, and waveform information.

## Patentansprüche

1. System, das konfiguriert ist, um eine Zweiwegekommunikation unter Verwendung eines Widerstandspegelkommunikationsprotokolls herzustellen, wobei das System umfasst:
einen Generator; und
eine elektrochirurgische Vorrichtung (300), umfassend:
einen elektrische Verbinder (302), der konfiguriert ist, um ein Behandlungssignal und ein Dauersignal von dem Generator zu empfangen;
einen Wechselstrom-Gleichstrom-Wandler (316), der konfiguriert ist, um das Dauersignal in ein Gleichstromsignal umzuwandeln;
einen Prozessor (218), der konfiguriert ist, um das Gleichstromsignal zu empfangen; und
einen Widerstand (320), der mit dem Prozessor (318) verbunden ist und konfiguriert ist, um einen von dem Prozessor (318) verwendeten Leistungsstrom im Laufe der Zeit zu variieren;
wobei der Generator konfiguriert ist, um Schwankungen im Strom als Daten zu interpretieren und der elektrochirurgischen Vorrichtung (300) unter Verwendung von Frequenzumtastung, FSK, eine Bestätigung bereitzustellen.

2. System nach Anspruch 1, wobei die Bestätigung auf den Daten basiert,
wobei, optional, die Bestätigung eine Ausgabe eines auf die Daten angewendeten Verschlüsselungsalgorithmus umfasst,
wobei, ferner optional, der Verschlüsselungsalgorithmus basierend auf mindestens einem Teil der Daten ausgewählt wird.

3. System nach Anspruch 1, wobei die Bestätigung aus einer festgelegten Anzahl von Bytes besteht.

4. System nach Anspruch 1, wobei die Daten auf mehreren Messwerten des Stroms innerhalb einer Zeitspanne basieren.

5. System nach Anspruch 1, wobei der Generator ferner konfiguriert ist, die Frequenz des Behandlungssignals basierend auf den Daten zu begrenzen.

6. System nach Anspruch 1, wobei der Generator ferner konfiguriert ist, um basierend auf den Daten eine Warnung bereitzustellen, dass die elektrochirurgische Vorrichtung (300) abgelaufen ist.

7. Verfahren zur Zweiwegekommunikation zwischen einer elektrochirurgischen Vorrichtung und einem Generator, umfassend:
Bereitstellen, über den Generator, der elektrochirurgischen Vorrichtung (300) und an der elektrochirurgischen Vorrichtung (300), eines Dauersignals:
Umwandeln des Dauersignals in ein Gleichstromsignal;
Versorgen eines Prozessors (318) mit dem Gleichstromsignal; und
Variieren, unter Verwendung eines Widerstands (320), des durch den Prozessor (318) verwendeten Leistungsstroms im Laufe der Zeit;
Bereitstellen, über die elektrochirurgische Vorrichtung (300), dem Generator und an dem Generator des Stroms:
Interpretieren von Schwankungen in dem Strom als Daten; und
Senden einer Bestätigung an die elektrochirurgische Vorrichtung (300) unter Verwendung von Frequenzumtastung, FSK.

8. Verfahren nach Anspruch 7, wobei die Daten eines oder mehreres von einer Vorrichtungs-ID, einer akkumulierten Nutzungszeit für die Vorrichtung, Herstellungsinformationen und Wellenforminformationen sind.

9. Verfahren nach Anspruch 7, wobei die Bestätigung auf den Daten basiert, wobei, optional, die Bestätigung die Ausgabe eines auf die Daten angewendeten Verschlüsselungsalgorithmus umfasst,
wobei, ferner optional, der Verschlüsselungsalgorithmus basierend auf mindestens einem Teil der Daten ausgewählt wird.

10. Verfahren nach Anspruch 7, wobei die Bestätigung eine festgelegte Anzahl von Bytes ist.

11. Verfahren nach Anspruch 7, wobei die Daten auf mehreren Messwerten des Stroms innerhalb einer Zeitspanne basieren.

12. Verfahren nach Anspruch 7, wobei der Generator ferner konfiguriert ist, um die Frequenz eines Behandlungssignals, das der elektrochirurgischen Vorrichtung (300) bereitgestellt wird, basierend auf den Daten zu begrenzen.

13. Verfahren nach Anspruch 7, ferner umfassend, dass der Generator basierend auf den Daten eine Warnung bereitstellt, dass die elektrochirurgische Vorrichtung (300) abgelaufen ist.

14. System, das konfiguriert ist, um eine Zweiwegekommunikation unter Verwendung eines Widerstandspegel-Kommunikationsprotokolls herzustellen, wobei das System umfasst:
einen Generator; und
eine elektrochirurgische Vorrichtung (200), umfassend:
einen elektrischen Verbinder (202), der konfiguriert ist, um ein Behandlungssignal und ein Dauersignal von dem Generator zu empfangen;
einen Transformator (216), der konfiguriert ist, um das Dauersignal zu isolieren;
ein digitales Potentiometer (218), das konfiguriert ist, um das isolierte Dauersignal zu empfangen; und
eine Steuerung (220), die konfiguriert ist, um den Widerstand des digitalen Potentiometers im Laufe der Zeit zu variieren;
wobei der Generator konfiguriert ist, um die Bewertung im Widerstand als Daten zu interpretieren und der elektrochirurgischen Vorrichtung (200) unter Verwendung von Frequenzumtastung, FSK, eine Bestätigung bereitzustellen.

15. System nach Anspruch 1 oder Anspruch 14, wobei die Daten eines oder mehreres von einer Vorrichtungs-ID, einer akkumulierten Nutzungszeit für die Vorrichtung, Herstellungsinformationen und Wellenforminformationen sind.

## Revendications

1. Système configuré destiné à établir une communication bidirectionnelle à l'aide d'un protocole de communication à niveau de résistance, le système comprenant :
un générateur ; et
un dispositif électrochirurgical (300) comprenant :
un connecteur électrique (302) configuré pour recevoir un signal de traitement et un signal continu du générateur ;
un convertisseur CA-CC (316) configuré pour convertir le signal continu en un signal CC ;
un processeur (218) configuré pour recevoir le signal CC ; et
une résistance (320) connectée au processeur (318) et configurée pour faire varier un courant d'alimentation utilisé par le processeur (318) dans le temps ;
dans lequel le générateur est configuré pour interpréter des variations du courant comme des données et fournir un accusé de réception au dispositif électrochirurgical (300) à l'aide d'une modulation par déplacement de fréquence, FSK.

2. Système selon la revendication 1, dans lequel l'accusé de réception est basé sur les données,
dans lequel, facultativement, l'accusé de réception comprend la sortie d'un algorithme de chiffrement appliqué aux données,
dans lequel, en outre éventuellement, l'algorithme de chiffrement est sélectionné en fonction d'au moins une partie des données.

3. Système selon la revendication 1, dans lequel l'accusé de réception est un nombre d'octets défini.

4. Système selon la revendication 1, dans lequel les données sont basées sur plusieurs lectures du courant
dans une période de temps.

5. Système selon la revendication 1, dans lequel le générateur est en outre configuré pour limiter la fréquence du signal de traitement sur la base des données.

6. Système selon la revendication 1, dans lequel le générateur est en outre configuré pour fournir une alerte indiquant que le dispositif électrochirurgical (300) a expiré sur la base des données.

7. Procédé de communication bidirectionnelle entre un dispositif électrochirurgical et un générateur comprenant :
la fourniture, par l'intermédiaire du générateur, d'un signal continu au dispositif électrochirurgical (300) et au niveau du dispositif électrochirurgical (300) :
la conversion du signal continu en un signal de courant continu ;
l'alimentation d'un processeur (318) avec le signal continu ; et
la variation, à l'aide d'une résistance (320), du courant d'alimentation utilisé par le processeur (318) dans le temps ;
la fourniture, par l'intermédiaire du dispositif électrochirurgical (300), du courant au générateur et au niveau du générateur :
l'interprétation des variations du courant en tant que données ; et
l'envoi d'un accusé de réception au dispositif électrochirurgical (300) à l'aide d'une modulation par déplacement de fréquence, FSK.

8. Procédé selon la revendication 7, dans lequel les données sont un ou plusieurs éléments parmi un identifiant de dispositif, un temps d'utilisation cumulé pour le dispositif, des informations de fabrication et des informations de forme d'onde.

9. Procédé selon la revendication 7, dans lequel l'accusé de réception est basé sur les données, dans lequel, éventuellement, l'accusé de réception comprend la sortie d'un algorithme de chiffrement appliqué aux données,
dans lequel, en outre éventuellement, l'algorithme de chiffrement est sélectionné en fonction d'au moins une partie des données.

10. Procédé selon la revendication 7, dans lequel l'accusé de réception est un nombre défini d'octets.

11. Procédé selon la revendication 7, dans lequel les données sont basées sur de multiples lectures du courant sur une période de temps.

12. Procédé selon la revendication 7, dans lequel le générateur est en outre configuré pour limiter la fréquence d'un signal de traitement fourni au dispositif électrochirurgical (300) sur la base des données.

13. Procédé selon la revendication 7, comprenant en outre la fourniture, par le générateur, d'une alerte indiquant que le dispositif électrochirurgical (300) a expiré sur la base des données.

14. Système configuré destiné à établir une communication bidirectionnelle à l'aide d'un protocole de communication à niveau de résistance, le système comprenant :
un générateur ; et
un dispositif électrochirurgical (200) comprenant :
un connecteur électrique (202) configuré pour recevoir un signal de traitement et un signal continu du générateur ;
un transformateur (216) configuré pour isoler le signal continu ;
un potentiomètre numérique (218) configuré pour recevoir le signal continu isolé ; et
un dispositif de commande (220) configuré pour faire varier la résistance du potentiomètre numérique dans le temps ;
dans lequel le générateur est configuré pour interpréter l'évaluation dans la résistance en tant que données et fournir un accusé de réception au dispositif électrochirurgical (200) à l'aide d'une modulation par déplacement de fréquence, FSK.

15. Système selon la revendication 1 ou la revendication 14, dans lequel les données sont une ou plusieurs parmi un identifiant de dispositif, un temps d'utilisation cumulé pour le dispositif, des informations de fabrication et des informations de forme d'onde.
